# FASCICULE DE BREVET EUROPEEN

(11) **EP 0 512 911 B1**
(45) Date de publication et mention de la délivrance du brevet: **09.08.1995**
(21) Numéro de dépôt: 92401253.7
(22) Date de dépôt: 04.05.1992
(51) Int. Cl.: C07C 11/02, C07C 5/333, B01J 8/06

(54) **Procédé et dispositif de déshydrogénation d'hydrocarbures aliphatiques en hydrocarbures oléfiniques**
Verfahren und Vorrichtung zur Dehydrierung von aliphatischen Kohlenwasserstoffen zu olefinischen Kohlenwasserstoffen
Process and apparatus for the dehydrogénation of aliphatic hydrocarbons into olefinic hydrocarbons

(30) Priorité: 06.05.1991 FR 9105671; 06.05.1991 FR 9105673
(43) Date de publication de la demande: 11.11.1992
(73) Titulaire: INSTITUT FRANCAIS DU PETROLE, 92502 Rueil-Malmaison (FR)
(72) Inventeur: Duée, Didier, F-95610 Eragny sur Oise (FR); Mank, Larry, F-78630 Orgeval (FR); Renard, Pierre, F-78860 Saint Nom La Breteche (FR); Burzynski, Jean-Pierre, F-69005 Lyon (FR); Leger, Gérard, F-69130 Ecully (FR); Vacher, Philippe, F-38200 Vienne (FR); Minkkinen, Ari, F-78860 Saint Nom La Breteche (FR)

(56) Documents cités:
- FR-A- 1 549 226
- US-A- 4 704 497
- PATENT ABSTRACTS OF JAPAN vol. 12, no. 482 (C-553)(3329) 15 Décembre 1988; & JP-A-63 197 534 (MITSUBISHI HEAVY IND) 16 Août 1988

## Description

Cette invention concerne un procédé et un dispositif de conversion d'hydrocarbures, notamment de production d'hydrocarbures oléfiniques, comportant au moins une double liaison, à partir d'une charge d'hydrocarbures aliphatiques de 2 à 20 atomes de carbone en présence d'une composition de catalyseur. Cette conversion met en jeu une réaction endothermique de production d'hydrocarbures suivie d'une réaction exothermique de régénération du catalyseur sur lequel s'est déposé du coke. L'invention concerne aussi l'utilisation du dispositif. Elle concerne plus particulièrement la synthèse d'isobutène qui est utilisé notamment pour la préparation du MTBE (méthyl tertio-butyl éther) en vue de l'amélioration de l'indice d'octane des essences.

La valorisation des coupes en particulier aliphatiques à bas point d'ébullition telles que la coupe C₄ de vapocraquage ou de craquage catalytique et les LPG, justifie l'intérêt que l'on peut porter à la mise en oeuvre de procédés de conversion de ces hydrocarbures qui soient performants, sélectifs et économiques tout en contribuant également à la formation d'hydrogène.

L'arrière plan technologique est illustré par le brevet JP-A-63197534.

La réaction de production d'hydrocarbures oléfiniques a été décrite, notamment dans le brevet US 4 704 497. Elle met en oeuvre un catalyseur comprenant de l'alumine.

Les processus élémentaires mis en jeu dans la transformation des hydrocarbures aliphatiques en hydrocarbures oléfiniques sont principalement la déshydrogénation des paraffines. Globalement, la réaction est endothermique, la vitesse de réaction est sensible aux variations de température et ces réactions successives s'accompagnent d'un dépôt de coke sur le catalyseur et d'une réduction des oxydes métalliques contenus dans le catalyseur, ce qui le désactive très rapidement et réduit la durée de cycle.

Un des problèmes à résoudre consiste donc à assurer une uniformité du chauffage de la zone réactionnelle aux environs de 500 à 600 °C permettant d'obtenir un profil de température le plus plat possible dans celle-ci, sachant de plus que le catalyseur est sensible à une augmentation de température et qu'il peut être détruit lorsque la température critique est dépassée.

En effet, on a remarqué que la demande en chaleur n'est pas constante au cours de l'avancement de la réaction endothermique de production d'hydrocarbures oléfiniques.

Par ailleurs, un autre problème a résoudre est relatif à la régénération du catalyseur qui doit être rapide, et de fréquence variable suivant la température de la réaction directement dépendante de la charge à traiter et donc suivant la quantité de coke déposé. En général, cette régénération est effectuée par exemple toutes les dix heures. Cette régénération doit être suffisamment douce afin de préserver les performances du catalyseur et minimiser son taux de renouvellement.

De plus, lors de la phase de régénération du catalyseur usagé, il est préférable, pour conserver le plus longtemps possible l'activité du catalyseur, d'atteindre le plus rapidement possible la température de combustion du coke et de maintenir un niveau de température sensiblement constant tout le long du tube et de ce fait un niveau d'activité homogène.

Il a été préconisé alors d'introduire les gaz de régénération à un niveau de température très élevé (600-700 °C) afin de ne pas trop refroidir le catalyseur à l'entrée du réacteur et de compenser le déficit en coke nécessaire à l'initiation de la réaction.

Il a aussi été préconisé d'ajouter un carburant gazeux ou liquide en quantité suffisante pour augmenter la température du gaz de régénération avant qu'il entre dans le réacteur.

Cette solution présente l'inconvénient d'initier le craquage des molécules d'hydrocarbures et de favoriser l'apparition de sous-produits indésirables.

L'objet de l'invention est de répondre aux problèmes soulevés ci-dessus de façon à améliorer les taux de conversion en hydrocarbures oléfiniques et la durée de vie du catalyseur.

Plus particulièrement, l'invention concerne un procédé de production d'hydrocarbures oléfiniques à partir d'une charge comprenant des hydrocarbures aliphatiques de 2 à 20 atomes de carbone susceptibles d'être déshydrogénés dans au moins une enceinte réactionnelle comportant une pluralité de tubes sensiblement parallèles, disposés en rangées et contenant un lit fixe de catalyseur, ledit procédé étant caractérisé en ce qu'on effectue :
a) une phase réactionnelle de production d'hydrocarbures oléfiniques au cours de laquelle on fait circuler ladite charge, éventuellement préchauffée, dans lesdits tubes contenant la composition catalytique dans des conditions appropriées et on recueille un effluent riche en hydrocarbures oléfiniques ;
b) une phase de purge des tubes avec au moins un gaz approprié après la phase réactionnelle et après une phase de régénération du catalyseur définie ci-dessous, et on recueille un effluent de purge ;
c) et une phase de régénération, dans lesdits tubes de l'enceinte, du catalyseur on lit fixe sur lequel s'est déposé du coke lors de la phase réactionnelle dans des conditions de régénération appropriées, et on récupère un effluent de régénération, ledit procédé étant en outre caractérisé en ce qu'on effectue, lors de la phase réactionnelle, le chauffage des tubes par une pluralité de moyens de chauffage radiants appropriés, disposés en nappes sensiblement parallèles, indépendantes entre elles, qui sont sensiblement perpendiculaires aux tubes, lesdits moyens de chauffage étant adaptés a chauffer une première partie (côté alimentation) des tubes avec un flux thermique supérieur au flux thermique moyen de l'enceinte réactionnelle nécessaire a la production d'hydrocarbures, et a chauffer une deuxième partie des tubes subséquents de la première avec un flux thermique au plus égal audit flux thermique moyen de telle façon que l'isothermicité du catalyseur soit sensiblement maintenue, et on évacue éventuellement de ladite enceinte des fumées de combustion provenant desdits moyens de chauffage.

Par charge, on entend un mélange d'hydrocarbures aliphatiques et d'hydrogène ou de vapeur d'eau.

Par hydrocarbures oléfiniques, on entend un hydrocarbure comportant au moins une double liaison.

Selon une caractéristique de procédé, on peut chauffer de 1 à 50 % de la longueur des tubes réactionnels, côté alimentation, avec un flux thermique compris entre 101 et 500 % du flux thermique moyen de l'enceinte réactionnelle et la partie restante des tubes avec un flux thermique compris entre 10 et 100 % dudit flux thermique moyen.

On définit le flux thermique moyen de l'enceinte par le rapport de la puissance absorbée par les tubes de l'enceinte pour une réaction considérée sur la somme de la surface extérieure de ces tubes.

De manière avantageuse, on peut chauffer de 1 a 40 % et de préférence de 1 à 35 % de la longueur des tubes réactionnels, côté alimentation avec un flux thermique compris entre 120 et 300 % et de préférence 150 à 200 % du flux thermique moyen et la partie restante des tubes avec un flux thermique compris entre 20 et 85 % et de préférence entre 40 et 75 % du flux thermique moyen.

Dans ces conditions, le profil de température tout le long du tube est sensiblement plat compte tenu d'une demande plus importante en chaleur dans la première partie du tube que dans la partie restante. Dans ce cas, l'utilisation du catalyseur peut être optimisée en relation avec le quasi maintien de son activité le plus longtemps possible.

Les moyens de chauffage utilisés peuvent avantageusement être ceux décrits dans le brevet US 4 664 620 et qui comprennent des brûleurs de forme sensiblement cylindrique, allongée à matrice en fibre de céramique qui brûlent, sans flamme, dans les espaces intersticiels entre les fibres, un mélange de combustible gazeux et d'air et qui transfèrent la chaleur par radiation. L'utilisation de ces brûleurs à fibre de céramique est donc un objet de l'invention.

Ils ont par ailleurs l'avantage de dégager lors de la combustion peu de Noₓ, peu de Co et d'hydrocarbure. Leur utilisation est par ailleurs très souple et d'une grande flexibilité, la quantité de chaleur dégagée pouvant être contrôlée et pouvant préchauffer la charge, les gaz de purge et de régénération.

De plus, ils sont d'une très faible inertie, ce qui est appréciable en cas d'un arrêt inopportun d'alimentation de la charge ou d'un dépôt de coke excessif sur le catalyseur pouvant interrompre l'alimentation de la charge.

Selon une autre caractéristique du procédé, on effectue habituellement une phase de purge entre la phase de production d'hydrocarbures oléfiniques et la phase de régénération du catalyseur usagé. Pour ce faire, on diminue ou on stoppe l'alimentation en la charge des tubes, et on purge au moins une fois les tubes avec un gaz inerte comme l'azote dans des conditions de débit et de température telles que la température du catalyseur reste sensiblement constante. Cette phase de purge pourra être reproduite entre une phase de régénération et une phase réactionnelle. De ce fait, le catalyseur est sous atmosphère de gaz inerte avant d'être mis en contact soit avec de l'oxygène lors de sa phase de régénération soit avec des hydrocarbures lors de la phase réactionnelle.

Durant la phase de purge, il est cependant préférable de pouvoir maintenir une température du catalyseur sensiblement constante tout le long des tubes réactionnels soit dans l'optique d'une régénération subséquente du catalyseur soit dans l'optique d'une phase de déshydrogénation de la charge hydrocarbonée. Cette température qui peut être contrôlée, sera de préférence la plus proche possible de la température de la phase de réaction et de celle de la phase de régénération afin d'éviter des discontinuités de profil qui altèrent les propriétés du catalyseur et qui fatiguent le matériau.

Par ailleurs, le maintien du chauffage durant la période de purge favorise la stabilité du préchauffage du gaz de la charge, de la purge ou de la régénération, au plus haut niveau possible.

Selon une autre caractéristique du procédé, on effectue en général la phase de régénération du catalyseur en injectant le gaz inerte, de préférence préchauffé et au moins un gaz contenant de 0,01 à 5 % en volume d'oxygène moléculaire et de préférence de 0,4 à 0,8 % en volume dans les tubes et on chauffe de 1 à 50 % et de préférence de 1 à 35 % de la longueur des tubes côté alimentation avec un flux thermique compris entre 5 et 100 % du flux thermique moyen de l'enceinte réactionnelle utilisé lors de la phase réactionnelle, dans des conditions telles que l'exothermicité de la réaction de combustion du coke est contrôlée et la température du catalyseur est maintenue sensiblement constante le long des tubes réactionnels.

La phase de régénération du catalyseur peut aussi être effectuée en plusieurs étapes successives :
a) une combustion à l'aide d'au moins un gaz renfermant de l'oxygène moléculaire dans les proportions indiquées ci-avant et à une température comprise en général entre 400 et 650 et avantageusement 500-550 °C, selon les conditions opératoires ci-avant.
b) une oxychloration au moyen d'un gaz renfermant de l'oxygène moléculaire et simultanément du chlore ou un composé chloré, de préférence en présence d'air humide, de chlore et d'azote à une température comprise en général entre 450 et 550 °C.
c) éventuellement, une calcination finale au moyen d'un gaz renfermant en général une concentration plus forte en oxygène notamment si on a réalisé l'oxychloration avec de l'air humide.

Bien évidemment, le passage de la phase de régénération à la phase de production d'hydrocarbures s'effectue en général par une phase de purge comme ci-dessus par exemple, qui amènera le catalyseur d'une atmosphère oxydante à une atmosphère inerte, suivie avantageusement d'une réduction sous H₂ avant le début de la phase réactionnelle.

Selon un mode de réalisation préféré du procédé selon l'invention, l'enceinte réactionnelle peut comprendre au moins un module comportant deux ensembles de tubes disposés en rangées sensiblement parallèles. On effectue en général la phase réactionnelle dans l'un de ces ensembles de tubes pendant que l'on effectue la phase de régénération du catalyseur dans l'autre ensemble de tubes. La régénération étant sensiblement terminée, après la période de purge, les tubes fonctionnant auparavant en régénération peuvent fonctionner alors en réacteur de production d'oléfines.

Ce fonctionnement en alternance par un jeu de vannes contrôlées par des moyens appropriés s'avère très souple.

L'homogénéité de température du lit de catalyseur durant la phase de production d'hydrocarbures est un avantage lors de sa régénération dans les mêmes tubes. Le profil de coke déposé tout le long du tube est sensiblement constant d'où une température de régénération plus uniforme.

Le procédé ainsi décrit permet de réduire le temps de passage entre le cycle de réaction et le cycle de régénération puisque la température est sensiblement la même dans les deux cas et puisqu'il est effectué dans les mêmes tubes.

La réaction de déshydrogénation s'effectue en général à une pression comprise entre 0,2 et 20 bar absolus (1 bar = 0,1 MPa) et à une température de 500 à 800 °C en fonction de la nature de la charge, la température étant avantageusement de 600 à 700 °C pour le propane et de 550 à 650 °C pour la coupe contenant de l'isobutane sous une pression préférée de 1 à 3 bar absolus. Les vitesses spatiales préconisées sont habituellement de 0,5 à 20 h⁻¹ et préférentiellement de 1,5 à 2,5 h⁻¹.

Le catalyseur peut être celui décrit par exemple dans le brevet US 4 806 624. Il peut comprendre par exemple un support amorphe ou cristallin, de préférence de l'alumine, ayant une surface spécifique de 25 à 500 m²/g environ.

Il comprend avantageusement au moins un métal noble du groupe VIII de la classification périodique des éléments, c'est-à-dire choisi dans le groupe formé par le ruthénium, le rhodium, le palladium, l'osmium, l'irridium et le platine.

Il comprend, en outre, avantageusement au moins un promoteur choisi parmi les éléments du groupe VII B, par exemple le manganèse et le rhénium, de préférence le rhénium ou du groupe IVA, de préférence l'étain.

Le catalyseur peut être neutralisé par au moins un composé comportant un élément alcalin ou alcalino-terreux, avantageusement le calcium, le strontium, le baryum et le radium, de préférence le potassium.

Le catalyseur choisi sera fonction de la charge à traiter.

Le catalyseur utilisé peut être aussi une zéolithe cristalline comme les mordénites naturelles ou de synthèse.

Ces zéolithes pourront contenir avantageusement au moins un métal cité ci-avant.

On peut également utiliser des zéolithes synthétisées en milieu fluorure avec ou sans métal, ce métal pouvant être dans la charpente ou hors charpente.

La charge d'hydrocarbures peut comprendre des hydrocarbures saturés ou insaturés avec une seule double liaison, avantageusement de 2 à 6 atomes de carbone. Ces hydrocarbures peuvent être du propane, du n-butane, du n-pentane, les isomères du butane et du pentane ou leurs mélanges. Ils peuvent être aussi par exemple du propène, des butènes et des pentènes et sont en général des produits résultant de la déshydrogénation de la charge d'origine, qui sont recyclés.

Ces hydrocarbures sont introduits purs ou dilués avec un gaz inerte dans le lit fixe.

A ces hydrocarbures, est associé, dans la charge, de l'hydrogène provenant d'un recyclage du produit final séparé, ou de la vapeur d'eau.

Le recyclage de l'hydrogène est tel que le rapport en mole H₂ sur hydrocarbures est compris entre 1 et 10, de préférence entre 2 et 5. De même, le rapport en mole H₂O sur hydrocarbures peut être compris entre 1 et 10 et de préférence entre 2 et 5.

On a obtenu d'excellents résultats avec une charge comprenant de l'hydrogène et de l'isobutane provenant d'une coupe C₄ de vapocraquage ou de craquage catalytique, ou bien d'une coupe LPG dans laquelle on trouve des butanes en grande quantité.

Par exemple, la charge peut avoir la composition suivante:
isobutane 20-93 %
n-butane 5-78 %
C₃ + C₅ 2-5%
Le dispositif selon l'invention permet ainsi de manière très rapide et par un contrôle aisé de la température de l'enceinte d'accepter des charges de composition variable. Il permet aussi d'ajuster le taux de régénération du catalyseur en fonction du bilan thermique et en fonction du taux de coke sur le catalyseur qui dépend notamment de la nature de la charge. Par ailleurs, un autre avantage est dû au fait que la charge, les gaz de purge et les gaz de régénération peuvent être préchauffés, notamment dans la section de convection des fumées de combustion des moyens de chauffage et introduits à une température adéquate.

De manière générale, les conditions opératoires sont optimisées de façon à convertir 10 à 70 % par passe, de la charge et de préférence 30-50 % avec une sélectivité en oléfines d'au moins 85 % et de préférence 90-95 %. Le rendement par passe en oléfines produites (iso et n) est généralement supérieur à 20 % et de préférence compris entre 30 et 50 % en poids.

On peut recycler, après séparation des effluents la partie de la charge non convertie.

Des taux de conversion plus importants peuvent être obtenus avec des charges plus lourdes ; par exemple au moins 95 %.

La régénération est généralement effectuée à une température comprise entre 400 et 650 °C et de préférence entre 500 et 550 °C.

Le procédé selon l'invention peut être mis en oeuvre dans un dispositif de conversion d'hydrocarbures comprenant au moins une enceinte (1) contenant au moins un réacteur (2) dans lequel est effectuée la réaction endothermique et qui comporte une pluralité de tubes (3) sensiblement parallèles entre eux et alimentés en parallèle, les tubes étant remplis d'au moins un catalyseur en lit fixe, des moyens (4) d'alimentation en une charge reliés à une extrémité desdits tubes et des moyens de récupération (5) d'un effluent connectés à l'autre extrémité desdits tubes, lesdits tubes étant disposés en rangées sensiblement parallèles et étant chauffés dans des conditions appropriées par une pluralité de moyens (6) de chauffage radiants tels que des brûleurs à fibre céramique, disposés en nappes sensiblement parallèles, indépendantes entre elles, qui sont sensiblement perpendiculaires aux tubes, lesdits moyens de chauffage comprenant une alimentation (7) en comburant et en carburant et une évacuation (16) des fumées de combustion reliée audit réacteur, ledit dispositif comportant en outre des moyens de régénération (9, 28) du catalyseur usagé comprenant des moyens de purge des tubes par au moins un gaz approprié, des moyens (9a, 9b) d'alimentation en au moins un gaz de régénération et des moyens d'évacuation (10, 32) de l'effluent de régénération, adaptés à régénérer le catalyseur usagé dans lesdits tubes, ces moyens de régénération étant connectés auxdits tubes, et des moyens (20) de changement en alternance adaptés à relier les tubes alternativement aux moyens (4) d'alimentation en charge et aux moyens (5) de récupération de l'effluent puis auxdits moyens de régénération (9, 28) du catalyseur, ledit dispositif comportant de plus des moyens (50) de contrôle et de régulation de la température des tubes connectés auxdites nappes des moyens de chauffage.

Les moyens de chauffage comprennent généralement une alimentation en carburant et en comburant et des moyens d'évacuation des fumées de combustion connectés par des passages appropriés à une chambre de convection, elle-même reliée à une cheminée. La chambre de convection peut comprendre un échangeur de chaleur adapté à préchauffer la charge, les gaz de purge et de régénération.

Les tubes utilisés dans le cadre du dispositif ont généralement une longueur de 2 à 20 m. Ils peuvent être cylindriques ou annulaires. Dans le cas de tubes annulaires, ils comprennent une partie annulaire périphérique contenant le catalyseur et une partie centrale.

Selon une première variante, la charge peut être introduite dans la partie annulaire à une extrémité, y circule axialement et l'effluent circule dans l'autre sens dans la partie centrale du tube où il est récupéré à l'autre extrémité située du même côté du tube. Dans ce cas, les tubes sont chauffés à leur périphérie par les moyens de chauffage.

Selon une deuxième variante préférée, la charge peut être introduite dans la partie annulaire à une extrémite, y circule axialement et est évacuée à l'extrémité opposée. La partie centrale de ces tubes est occupée partiellement par les moyens de chauffage de forme avantageusement cylindrique, jointifs ou séparés, adaptés à chauffer selon le principe énoncé ci-avant l'intérieur des tubes c'est-à-dire la partie annulaire contenant le catalyseur, l'alimentation de ces brûleurs et l'évacuation des fumées de combustion s'effectuant du même côté, côté sortie de l'effluent hydrocarboné. La distance entre les brûleurs et la paroi interne des tubes est habituellement comprise entre 0,2 et 10 fois l'épaisseur annulaire contenant le catalyseur. Ces tubes sont avantageux en raison de leur grande capacité et de leur compacité dans les réacteurs.

Dans le cas de tubes cylindriques, leur diamètre interne est de 10 mm à 200 mm. Ils sont disposés en rangées sensiblement parallèles et avantageusement verticales. Chaque rangée peut contenir une ou plusieurs lignes de tubes. La distance entre l'axe de chaque tube est en général comprise entre 1,5 et 6 fois son diamètre externe et les brûleurs sont en règle générale disposés à la périphérie des tubes.

Selon une autre caractéristique, le dispositif comprend habituellement des moyens de régénération adaptés à régénérer le catalyseur usagé dans les mêmes tubes où s'est déroulée la réaction de production d'oléfines. Ces moyens de régénération comportent en général une alimentation en gaz de régénération à l'une des extrémités du faisceau de tubes et une évacuation de l'effluent de régénération à l'autre extrémité, cette extrémité pouvant se trouver du même côté, par exemple avec des tubes annulaires.

Le dispositif peut comporter également des moyens adaptés à relier les tubes réactionnels alternativement aux moyens de régénération puis aux moyens nécessaires à la réalisation de la réaction, en particulier à relier une extrémité des tubes aux moyens d'alimentation en charge et l'autre extrémité aux moyens d'évacuation de l'effluent produit, cette extrémité pouvant se trouver du même côté dans le cas de tubes annulaires.

Selon un mode de réalisation préféré, le dispositif peut comprendre au moins une enceinte avec un premier réacteur contenant une pluralité de tubes réactionnels et un deuxième réacteur comportant une pluralité de tubes de régénération adaptés à régénérer le catalyseur, le premier et le second réacteur communiquant par au moins un passage (14, 15) avec une section de convection reliée à l'extérieur par des moyens appropriés (cheminée), ces tubes de régénération étant reliés à une extrémité à une alimentation en gaz de régénération et à l'autre extrémité à une évacuation d'un effluent de régénération et comprenant en outre les moyens de changement en alternance adaptés à relier les tubes réactionnels alternativement aux moyens d'alimentation en charge et aux moyens de récupération de l'effluent, puis à l'alimentation en gaz de régénération et à l'évacuation de l'effluent de régénération, ces moyens de changement en alternance étant adaptés à relier en outre les tubes de régénération alternativement à l'alimentation en gaz de régénération et à l'évacuation de l'effluent de régénération, puis aux moyens d'alimentation, en charge et aux moyens de récupération de l'effluent d'hydrocarbures oléfiniques, les tubes réactionnels opérant en phase dite réactionnelle pendant que les tubes de régénération opèrent en phase dite de régénération dans un premier temps et les tubes réactionnels devenant ensuite des tubes de régénération tandis que les tubes de régénération deviennent des tubes réactionnels dans un deuxième temps.

La technologie de conception très souple grâce à l'aspect modulaire de sa mise en oeuvre peut être adaptée aussi bien aux petites qu'aux grandes capacités, la souplesse et la flexibilité d'utilisation du chauffage dans de courts délais étant un atout supplémentaire.

Selon une autre caractéristique du dispositif, les moyens de contrôle et de régulation peuvent comprendre au moins une sonde de température reliée à ladite première partie des tubes et au moins une autre sonde de température connectée à la seconde partie subséquente des tubes, ces deux sondes délivrant des signaux, des moyens de traitement et de commande adaptés à recevoir les signaux, à les comparer à des valeurs de référence et à transmettre des informations par d'autre signaux susceptibles d'asservir les moyens de chauffage des premières et deuxièmes parties des tubes.

L'invention sera mieux comprise au vu des figures ci-dessous illustrant de manière schématique le procédé et le dispositif selon l'invention, parmi lesquelles :
- la figure 1 montre une coupe longitudinale de l'enceinte réactionnelle contenant une section réactionnelle et une section de régénération séparée par une chambre de convection ;
- la figure 2 représente les moyens de chauffage des tubes ainsi que les moyens de contrôle et de régulation qui les asservissent.

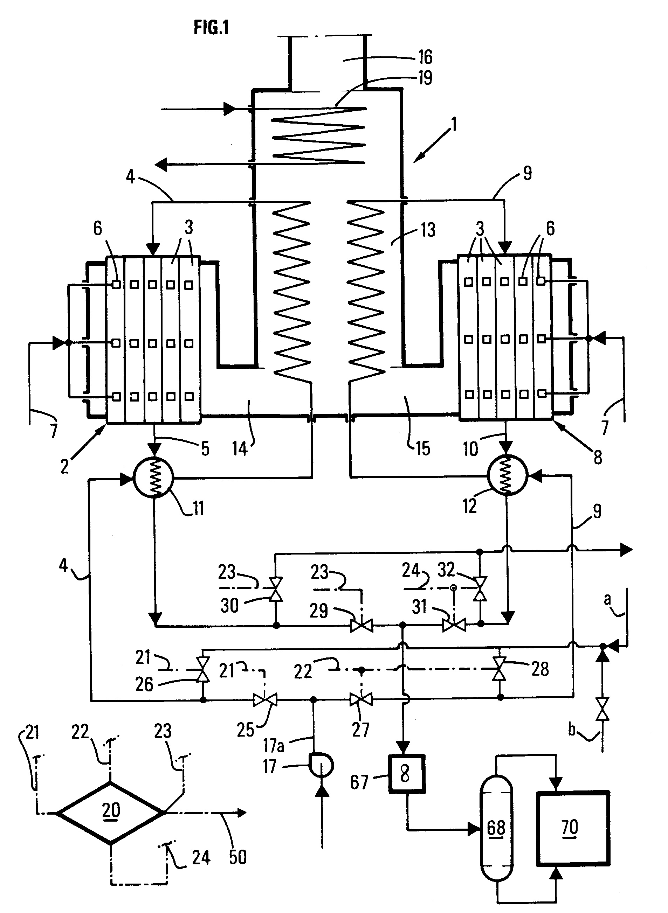

Selon la figure 1, une enceinte réactionnelle 1 ayant des parois revêtues d'un matériau isolant comprend un réacteur 2 comportant une pluralité de tubes 3 en acier inox de forme sensiblement cylindrique qui contiennent un lit fixe d'un catalyseur adapté à la production d'hydrocarbures mono ou dioléfiniques. Ces tubes distants les uns des autres d'une longueur d'environ 2 fois leur diamètre, mesurée à partir de l'axe, sensiblement verticaux sont disposés en rangées sensiblement parallèles contenant deux lignes de tubes, de préférence décalés. Entre les rangées sont situés une pluralité de brûleurs 6 de forme sensiblement cylindrique allongée à matrice en fibre de céramique brûlant sans flamme un mélange de combustible gazeux et d'air apporté par une alimentation 7. Ces brûleurs sont disposés en nappes sensiblement parallèles, indépendantes entre elles qui sont sensiblement perpendiculaires aux tubes réactionnels. Les nappes peuvent comprendre un ou plusieurs brûleurs et la distance entre brûleurs est généralement comprise entre 0,5 et 2,0 m. Par ailleurs, la distance entre brûleurs et tubes est généralement comprise entre 0,2 et 0,8 m, avantageusement entre 0,3 et 0,5 m. Ces brûleurs radiants sont adaptés à chauffer une première partie des tubes (côté alimentation) avec un flux thermique d'environ 180 % du flux thermique moyen de l'enceinte sur une longueur d'environ 30 % de la longueur des tubes qui peut atteindre 8 m. La partie restante des tubes est chauffée avec un flux thermique égal à environ 70 % du flux thermique moyen. Dans ces conditions, la réaction peut démarrer instantanément et il n'est pas nécessaire de trop préchauffer la charge qui risquerait de se craquer.

Les fumées de combustion des brûleurs sont évacuées par un passage 14 vers une section de convection 13 dans laquelle la charge peut être préchauffée. Un récupérateur de chaleur 19 peut aussi y être incorporé. Les fumées sont évacuées par une cheminée 16.

Les tubes du réacteur sont alimentés en parallèle par une charge comprenant des hydrocarbures aliphatiques, une coupe C₄ par exemple, amenée par des lignes 17a et 4, grâce à une pompe 17. La charge comprend aussi de l'hydrogène recyclé amenée par une ligne 17b. Cette charge peut être aussi préchauffée par un échangeur thermique 11 en amont de son passage dans la section de convection, cet échangeur recevant de la chaleur de l'effluent du réacteur qui est évacué, en parallèle par une ligne 5 reliée à l'échangeur. L'effluent est ensuite dirigé par un jeu de vannes appropriées 29 vers un aéro-réfrigérant 67 puis vers un ballon séparateur 68 de gaz et de liquide et enfin vers une section de fractionnement 70 permettant de recueillir les hydrocarbures produits par une ligne 71, un gaz enrichi en hydrogène qui peut être recyclé par une ligne 17b ainsi que la charge non convertie qui peut être recyclée.

L'enceinte réactionnelle comprend selon la figure 1 décrite ci-dessus une cellule de réaction. Elle comprend aussi une cellule de régénération 8 disposée de manière sensiblement symétrique par rapport à l'axe de l'enceinte passant par la section de convection. En fait, cette cellule de régénération est sensiblement identique à la cellule de production d'hydrocarbures puisque les deux réacteurs opèrent de manière alternée, l'une opérant en phase de production d'hydrocarbures pendant que l'autre opère en phase de régénération du catalyseur.

La cellule 8 opérant en régénération comprend donc la pluralité de tubes 3 décrits ci-dessus disposés en rangées entre lesquelles sont situés les brûleurs 6 disposés en nappes et qui sont adaptées à chauffer les tubes dans des conditions de régénération décrites ci-avant. Les gaz de combustion des brûleurs 6 sont dirigés par un passage 15 vers la section de convection 13. Ces tubes contiennent le catalyseur sur lequel s'est déposé du coke durant la phase précédente de production d'hydrocarbures. Tout d'abord, le gaz utilisé pour la purge (l'azote) amené par la ligne 9a, puis un mélange d'azote et d'air (0,5 % d'oxygène en volume) amené par les lignes 9a et 9b sont introduits après avoir été comprimés, à l'entrée du régénérateur 8 par la ligne 9 et la vanne 28. L'alimentation des tubes s'effectue en parallèle et dans la même direction que celle de l'introduction de la charge. Le catalyseur en lit fixe est au moins partiellement régénéré dans des conditions appropriées et l'effluent de combustion est évacué par une ligne 10 et traverse l'échangeur thermique 12 qui préchauffe partiellement le gaz de purge et le gaz de régénération. Un préchauffage supplémentaire de ces gaz est réalisé également dans la section de convection 13.

L'effluent de régénération est ensuite dirigé grâce à un jeu de vannes 32 vers une section de traitement et de séparation non représentée, par la ligne 10.

Selon le mode de réalisation déjà décrit, une cellule opère en production d'hydrocarbures oléfiniques pendant que l'autre opère en phase de régénération. Après le temps nécessaire à la réaction de production d'hydrocarbures (12 heures environ), des moyens de changement d'alternance appropriés 20 permettent d'alterner, le réacteur 2 passant en phase de régénération tandis que le régénérateur 8 passe en phase de production d'hydrocarbures.

Pendant la phase nécessaire à la purge, des vannes 26 et 28 alimentant l'ensemble du circuit en azote sont ouvertes, les vannes 25 et 27 étant fermées et des vannes 30 et 32 pour la récupération de l'effluent de purge sont ouvertes pendant que les vannes 29 et 31 sont fermées. Une phase de réduction du catalyseur sous H₂ peut être effectuée après sa régénération et la phase de purge.

Après la phase de purge, les moyens de changement d'alternance ouvrent la vanne 25 pour l'alimentation du réacteur en la charge comprenant l'hydrogène recyclé et ferment les vannes 26 et 27. Ces mêmes moyens ferment les vannes 30 et 31 pour laisser évacuer l'effluent par la vanne 29 ouverte vers la section de fractionnement 70.

De même, les moyens de changement d'alternance 20 laissent passer le mélange N₂ et air par la vanne 28 ouverte vers la cellule de régénération 8, la vanne 27 étant fermée tandis que l'effluent de régénération est dirigé vers la section de traitement grâce à la vanne 32 ouverte et la vanne 31 fermée.

Après une nouvelle période de purge, les moyens de changement 20 font passer le réacteur 2 en régénérateur, la ligne 9 du mélange de gaz de régénération alimente le réacteur par la vanne 26 ouverte, les vannes 28 et 25 étant fermées tandis que l'effluent de régénération est évacué par la ligne 5, la vanne 30 étant ouverte, les vannes 29 et 32 étant fermées. De même, les moyens de changement en alternance font passer le régénérateur 8 en réacteur. A cette fin, la charge est envoyée par la ligne 17b et la vanne 27 ouverte, les vannes 25 et 28 étant fermées, vers la ligne 9 alimentant les tubes réactionnels et les effluents de la réaction de production d'hydrocarbures oléfiniques sont envoyés vers la section de traitement 70 par la ligne 10, la vanne 31 étant ouverte et les vannes 29 et 32 étant fermées.

Bien entendu, les moyens de changement en alternance 20 sont connectés aux moyens 50 de régulation des nappes de chauffage qui adaptent le chauffage et donc l'alimentation en combustible de manière différente au moment des phases de production d'oléfines, de purge et de régénération.

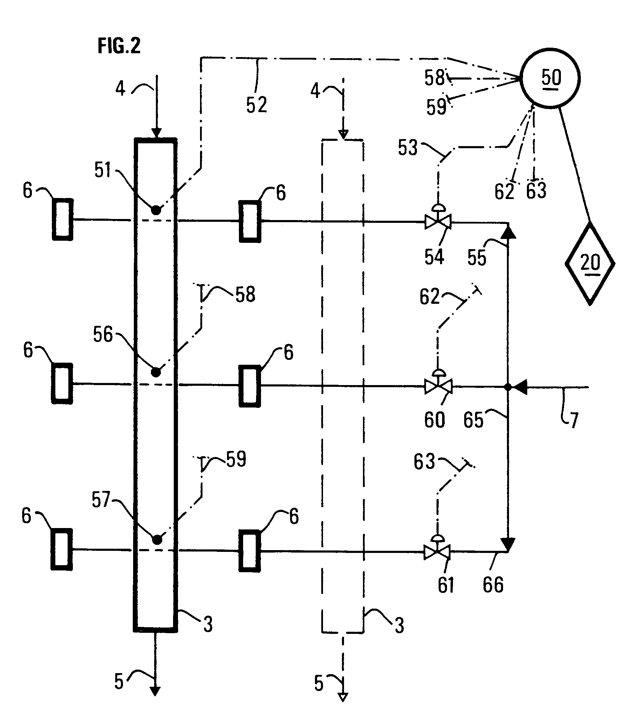

Les moyens de chauffage du réacteur 2 ou du réacteur 8 sont régulés et contrôlés de la manière suivante (figure 2).

Des sondes de température 51, 56 et 57 disposés sur les tubes réactionnels sensiblement au niveau des différentes nappes de chauffage 6, mesurent la température et envoient un signal électrique respectivement par des lignes électriques 52, 58 et 59 vers les moyens de contrôle et de régulation 50 eux-mêmes connectés aux moyens de changement en alternance 20. Pour une phase déterminée de réaction de production d'oléfines par exemple, les signaux électriques délivrés par les sondes sont comparés à des valeurs de référence préalablement enregistrées. Les moyens 50 envoient alors des signaux de réponse par les lignes 53, 62 et 63 qui régulent respectivement les vannes d'alimentation en combustible 54, 60 et 61 amené, par les lignes 55, 65 et 66 connectées à leur tour à la ligne d'alimentation en combustible 7, ces vannes alimentant en fonction des valeurs de référence et de la température mesurée les moyens de chauffage 6.

On a représenté une enceinte avec un seul module contenant un réacteur ou cellule opérant en phase de production d'hydrocarbures oléfiniques et un autre réacteur opérant en phase de régénération, ces deux réacteurs étant réunis par une section de convection grâce aux passages 14 et 15. Bien entendu, cette enceinte peut contenir plusieurs modules, les deux sections de convection adjacentes ayant par exemple une seule cheminée d'évacuation des effluents de combustion.

Par ailleurs, il a été précisé que la régénération du catalyseur pouvait comprendre une étape d'oxychloration après l'étape de combustion, qui sera effectuée avant l'étape de purge des tubes réactionnels précédant l'étape de production d'hydrocarbures oléfiniques.

De plus, l'étape de production d'hydrocarbures et l'étape de régénération sont effectuées, comme le montre la figure 1, en introduisant la charge et le gaz de régénération dans la même direction (du haut vers le bas par exemple) mais il est tout à fait possible d'introduire la charge dans une direction et le gaz de régénération dans une direction opposée, la modification du dispositif entrant dans l'esprit de l'invention.

## Revendications

1. Procédé de production d'hydrocarbures oléfiniques à partir d'une charge comprenant des hydrocarbures aliphatiques de 2 à 20 atomes de carbone susceptibles d'être déshydrogénés dans au moins une enceinte réactionnelle comportant une pluralité de tubes sensiblement parallèles, disposés en rangées et contenant un lit fixe de catalyseur, dans lequel on effectue :
a) une phase réactionnelle de production d'hydrocarbures oléfiniques au cours de laquelle on fait circuler ladite charge, éventuellement préchauffée, dans lesdits tubes contenant la composition catalytique dans des conditions appropriées et on recueille un effluent riche en hydrocarbures oléfiniques ;
b) une phase de purge des tubes avec au moins un gaz approprié après la phase réactionnelle et après une phase de régénération du catalyseur définie ci-dessous, et on recueille un effluent de purge ;
c) et une phase de régénération, dans lesdits tubes de l'enceinte, du catalyseur en lit fixe sur lequel s'est déposé du coke lors de la phase réactionnelle dans des conditions de régénération appropriées, et on récupère un effluent de régénération,
ledit procédé étant caractérisé en ce qu'on effectue, lors de la phase réactionnelle, le chauffage des tubes par une pluralité de moyens de chauffage radiants appropriés, disposés en nappes sensiblement parallèles, indépendantes entre elles, qui sont sensiblement perpendiculaires aux tubes, lesdits moyens de chauffage étant adaptés à chauffer une première partie (côté alimentation) des tubes représentant 1 à 50% de la longueur des tubes avec un flux thermique compris entre 101 et 500% du flux thermique moyen de l'enceinte réactionnelle, et à chauffer la partie restante des tubes avec un flux thermique compris entre 10 et 100% du flux thermique moyen de telle façon que l'isothermicité du catalyseur soit sensiblement maintenue, et on évacue éventuellement de ladite enceinte des fumées de combustion provenant desdits moyens de chauffage.

2. Procédé selon la revendication 1 dans lequel on chauffe de 1 à 40% et de préférence de 1 à 35 % de la longueur des tubes réactionnels côté alimentation avec un flux thermique compris entre 120 et 300 % et de préférence 150 à 200 % dudit flux thermique moyen et la partie restante des tubes réactionnels avec un flux thermique compris entre 20 et 85 % et de préférence entre 40 et 75 % du flux thermique moyen.

3. Procédé selon l'une des revendications 1 à 2 dans lequel, entre chaque phase réactionnelle et chaque phase de régénération et entre chaque phase de régénération et chaque phase réactionnelle, on stoppe l'alimentation respectivement en la charge et en gaz de régénération ; on stoppe éventuellement l'alimentation des brûleurs en carburant et en comburant et on purge au moins une fois les tubes réactionnels avec un gaz inerte tel que l'azote dans des conditions de débit et de température telles que la température du catalyseur est sensiblement constante.

4. Procédé selon l'une des revendications 1 à 3 dans lequel l'enceinte réactionnelle comporte au moins un module de deux ensembles de tubes, caractérisé en ce qu'on effectue la phase réactionnelle dans un de ces ensembles de tubes pendant que l'on effectue la phase de régénération du catalyseur dans l'autre ensemble de tubes et vice versa.

5. Procédé selon l'une des revendications 1 à 4 caractérisé en ce que la phase de régénération du catalyseur comporte l'injection d'un gaz inerte tel que l'azote, éventuellement préchauffé, et d'au moins un gaz contenant de 0,01 à 5 % d'oxygène moléculaire en volume dans lesdits tubes réactionnels et le chauffage de 1 à 50 % et de préférence de 1 à 35 % de la longueur des tubes réactionnels côté alimentation avec un flux thermique compris entre 5 et 100 % du flux thermique moyen de l'enceinte dans des conditions telles que l'exothermicité de la réaction de combustion du coke est contrôlée et la température du catalyseur est sensiblement constante.

6. Procédé selon l'une des revendications 1 à 5 dans lequel on effectue une étape d'oxychloration au moyen d'un gaz contenant de l'oxygène moléculaire et du chlore ou d'un composé chloré à une température de 450 à 550°C, puis éventuellement une étape de calcination avec une concentration plus élevée en oxygène que celle décrite dans la revendication 6.

7. Procédé selon l'une des revendications 1 à 6 dans lequel la charge comprend de l'hydrogène frais ou recyclé.

8. Procédé selon une des revendications 1 à 7 dans lequel la charge comprend de la vapeur d'eau.

9. Procédé selon l'une des revendications 1 à 8 dans lequel les moyens de chauffage sont des brûleurs à matrice en fibre de céramique.

10. Procédé selon l'une des revendications 1 à 9 dans lequel on contrôle et on régule l'isothermicité du catalyseur par des moyens de traitement et de commande connectés aux moyens de chauffage radiants et à au moins une première sonde de température reliée à la première partie des tubes et a au moins une seconde sonde reliées à la partie restante des tubes.

11. Dispositif de conversion d'hydrocarbures comprenant au moins une enceinte (1) contenant au moins un réacteur (2) dans lequel est effectué une réaction endothermique et qui comporte une pluralité de tubes (3) sensiblement parallèles entre eux et alimentés en parallèle, les tubes étant remplis d'au moins un catalyseur en lit fixe, des moyens (4) d'alimentation en une charge reliés à une extrêmité desdits tubes et des moyens de récupération (5) d'un effluent d'hydrocarbures connectés à l'autre extrêmité desdits tubes, lesdits tubes étant disposés en rangées sensiblement parallèles et étant chauffés dans des conditions appropriées par une pluralité de moyens (6) de chauffage, lesdits moyens de chauffage comprenant une alimentation (7) en comburant et en carburant et une évacuation (16) des fumées de combustion reliée audit réacteur, ledit dispositif comportant en outre des moyens de régénération (9, 28) du catalyseur usagé comprenant des moyens de purge des tubes par au moins un gaz approprié, des moyens (9a, 9b) d'alimentation en au moins un gaz de régénération et des moyens d'évacuation (10, 32) de l'effluent de régénération, adaptés à régénérer le catalyseur usagé dans lesdits tubes, ces moyens de régénération étant connectés auxdits tubes, et des moyens (20) de changement en alternance adaptés à relier les tubes alternativement aux moyens (4) d'alimentation en charge et aux moyens (5) de récupération de l'effluent puis auxdits moyens de régénération (9, 28) du catalyseur, le dispositif étant caractérisé en ce qu'il comporte des moyens de chauffage radiants tels que des brûleurs à fibre céramique, disposés en nappes sensiblement parallèles, indépendantes entre elles, qui sont sensiblement perpendiculaires aux tubes et en ce qu'il comporte en outre des moyens (50) de contrôle et de régulation de la température des tubes connectés auxdites nappes des moyens de chauffage et adaptés à chauffer une première partie des tubes côté alimentation avec un flux thermique supérieur au flux thermique moyen de l'enceinte et à chauffer la partie restante des tubes avec un flux thermique au plus égal au flux thermique moyen de l'enceinte.

12. Dispositif selon la revendication 11 dans lequel la longueur des tubes est de 2 à 20 m et leur diamètre interne de 10 à 200 mm.

13. Dispositif selon l'une des revendications 11 à 12 dans lequel la distance entre axe de chaque tube est comprise entre 1,5 et 6 fois son diamètre externe.

14. Dispositif selon l'une des revendications 11 à 13 dans lequel les moyens de contrôle et de régulation comprennent au moins une sonde de température reliée à une première partie des tubes côté alimentation et au moins une autre sonde reliés à une deuxième partie subséquente des tubes, ces deux sondes délivrant des signaux, des moyens de traitement et de commande adaptés à recevoir les signaux, à les comparer à des signaux de référence et à transmettre d'autres signaux susceptibles d'asservir les moyens de chauffage de première et deuxième parties des tubes.

15. Dispositif selon l'une des revendications 11 à 14 dans lequel la distance entre lesdits moyens de chauffage est comprise entre 0,5 et 2,0 m.

16. Dispositif selon l'une des revendications 11 à 15 dans lequel ladite enceinte comprend un premier réacteur contenant une pluralité des tubes réactionnels et un deuxième réacteur comportant une pluralité de tubes de régénération adaptés à régénérer le catalyseur, le premier et le second réacteur communiquant par au moins un passage (14, 15), avec une chambre (13) de convection reliée à l'extérieur par des moyens appropriés (cheminée), ces tubes de régénération étant reliés à une extrêmité à une alimentation en gaz de régénération et à l'autre extrêmité à une évacuation d'un effluent de régénération et comprenant en outre des moyens adaptés à relier les tubes réactionnels alternativement aux moyens d'alimentation en charge et aux moyens de récupération de l'effluent, puis à l'alimentation en gaz de régénération et à l'évacuation de l'effluent de régénération, ces moyens étant adaptés à relier en outre les tubes de régénération alternativement à l'alimentation en gaz de régénération et à l'évacuation de l'effluent de régénération, puis aux moyens d'alimentation en charge et aux moyens de récupération de l'effluent d'hydrocarbures, les tubes réactionnels opérant en phase dite réactionnelle pendant que les tubes de régénération opèrent en phase dite de régénération dans un premier temps et les tubes réactionnels devenant ensuite des tubes de régénération tandis que les tubes de régénération deviennent des tubes réactionnels dans un deuxième temps.

17. Dispositif selon la revendication 16 dans lequel la chambre de convection 13 comprend un échangeur de chaleur adapté à préchauffer la charge, les gaz de régénération et le gaz de purge.

18. Dispositif selon l'une des revendications 11 à 17 dans lequel les tubes réactionnels et de régénération sont chauffés par lesdits moyens de chauffage disposés à l'extérieur de ceux-ci.

19. Dispositif selon l'une des revendications 11 à 18 dans lequel les tubes comprennent une partie annulaire contenant le catalyseur et une partie centrale cylindrique à l'intérieur de laquelle sont logés lesdits moyens de chauffage.

20. Utilisation du dispositif selon l'une des revendications 11 à 19 dans un procédé de production d'hydrocarbures oléfiniques à partir d'une charge comprenant au moins un hydrocarbure déshydrogénable de 2 à 20 atomes de carbone.

## Claims

1. A method of producing olefinic hydrocarbons from a charge including aliphatic hydrocarbons with 2 to 20 carbon atoms which can be dehydrogenated in at least one reaction chamber, the chamber having a plurality of substantially parallel tubes arranged in rows and containing a fixed bed of catalyst, wherein:
a) a reaction phase producing olefinic hydrocarbons is carried out, during which the possibly preheated charge is circulated in the tubes containing the catalytic composition under appropriate conditions, and an effluent rich in olefinic hydrocarbons is collected;
b) a phase of purging the tubes with at least one appropriate gas is carried out, after the reaction phase and after a catalyst-regenerating phase defined below, and a purge effluent is collected;
c) and a phase of regenerating the catalyst is carried out in the tubes of the chamber under appropriate regenerating conditions, the catalyst being in a fixed bed and having had coke deposited on it during the reaction phase, and a regeneration effluent is recovered;
characterised in that the tubes are heated during the reaction phase by a plurality of appropriate radiant heating means arranged in layers which are substantially parallel, independent of one another and substantially perpendicular to the tubes, the heating means being adapted to heat a first part (the feed side) of the tubes, representing 1 to 50% of the length of the tubes, with a heat flow equal to 101 to 500% of the mean heat flow of the reaction chamber, and adapted to heat the remaining part of the tubes with a heat flow equal to 10 to 100% of the mean heat flow, so that the isothermicity of the catalyst is substantially maintained, and any combustion fumes emanating from the heating means are possibly discharged from the chamber.

2. The method of claim 1, wherein from 1 to 40% and preferably 1 to 35% of the length of the reaction tubes at the feed side is heated with a heat flow from 120 to 300% and preferably 150 to 200% of the mean heat flow, and the remaining part of the reaction tubes with a heat flow from 20 to 85% and preferably 40 to 75% of the mean heat flow.

3. The method of claim 1 or 2, wherein the charge feed and the regenerating gas feed are respectively stopped between each reaction phase and each regeneration phase and between each regeneration phase and each reaction phase, and wherein the fuel and oxidant feed to the burners may be stopped, and the reaction tubes are purged at least once with an inert gas such as nitrogen, under flow and temperature conditions such that the temperature of the catalyst is substantially constant.

4. The method of any of claims 1 to 3, wherein the reaction chamber comprises at least one module of two sets of tubes, characterised in that the reaction phase is carried out in one of these sets of tubes while the catalyst-regenerating phase is carried out in the other set of tubes and vice versa.

5. The method of any of claims 1 to 4, characterised in that the catalyst-regenerating phase comprises injecting an inert gas such as nitrogen, which is possibly preheated, and at least one gas containing from 0.01 to 5% of molecular oxygen by volume into said reaction tubes, and heating from 1 to 50% and preferably 1 to 35% of the length of the reaction tubes at the feed side with a heat flow equal to 5 to 100% of the mean heat flow of the chamber, under conditions such that the exothermicity of the coke combustion reaction is controlled and the temperature of the catalyst is substantially constant.

6. The method of any of claims 1 to 5, wherein an oxychlorination stage is carried out by means of a gas containing molecular oxygen and chlorine or a chlorine compound at a temperature from 450 to 550°C, possibly followed by a calcination stage with a higher concentration of oxygen that that described in claim 5.

7. The method of any of claims 1 to 6, wherein the charge comprises fresh or recycled hydrogen.

8. The method of any of claims 1 to 7, wherein the charge comprises water vapour.

9. The method of any of claims 1 to 8, wherein the heating means are burners with a ceramic fibre matrix.

10. The method of any of claims 1 to 9, wherein the isothermicity of the catalyst is checked and regulated by processing and control means connected to the radiant heating means, to at least one first temperature probe connected to the first part of the tubes and to at least one second probe connected to the remaining part of the tubes.

11. A hydrocarbon converting apparatus provided with at least one chamber 1 containing at least one reactor 2, in which an endothermic reaction takes place and which has a plurality of tubes 3, substantially parallel with one another and fed in parallel, the tubes being filled with at least one catalyst in a fixed bed, charge- feeding means 4 connected to one end of the tubes and hydrocarbon effluent- recovering means 5 connected to the other end of the tubes, the tubes being disposed in substantially parallel rows and being heated under appropriate conditions by a plurality of heating means 6, the heating means having means 7 for feeding fuel and oxidant and means 16 for discharging combustion fumes connected to said reactor, the apparatus further comprising means 9, 28 for regenerating the spent catalyst, including means for purging the tubes with at least one appropriate gas, means 9a, 9b for feeding at least one regenerating gas and means 10, 32 for discharging the regeneration effluent, adapted to regenerate the spent catalyst in the tubes, these regenerating means being connected to the tubes, and alternate displacement means 20 adapted to connect the tubes alternately to the charge feeding means 4 and effluent recovery means 5, then to the catalyst-regenerating means 9, 28, characterised in that it comprises radiant heating means such as ceramic fibre burners arranged in layers which are substantially parallel, independent of one another and substantially perpendicular to the tubes, and that it further comprises means 50 for controlling and regulating the temperature of the tubes, connected to the layers of heating means and adapted to heat a first part of the tubes at the feed side with a heat flow greater than the mean heat flow of the chamber, and to heat the remaining part of the tubes with a heat flow no more than equal to the mean heat flow of the chamber.

12. The apparatus of claim 11, wherein the length of the tubes is from 2 to 20 m and their inside diameter is from 10 to 200 mm.

13. The apparatus of claim 11 or 12, wherein the distance between the axis of each tube is from 1.5 to 6 times its outside diameter.

14. The apparatus of any of claims 11 to 13, wherein the checking and regulating means comprise at least one temperature probe connected to a first part of the tubes at the feed side and at least one other probe connected to a second, subsequent part of the tubes, the two probes emitting signals, and processing and control means adapted to receive the signals, compare them with reference signals and transmit other signals, which can control the means for heating the first and second parts of the tubes.

15. The apparatus of any of claims 11 to 14, wherein the distance between the heating means is from 0.5 to 2.0 m.

16. The apparatus of any of claims 11 to 15, wherein the chamber comprises a first reactor containing a plurality of reaction tubes and a second reactor provided with a plurality of regenerating tubes adapted to regenerate the catalyst; the first and second reactor communicating through at least one passage 14, 15 with a convection chamber 13 which is connected to the outside by appropriate means (chimney); the regenerating tubes being connected at one end to a regenerating gas feed and at the other end to means for discharging a regeneration effluent; and further comprising means adapted to connect the reaction tubes alternately to the charge-feeding means and effluent-recovery means then to the regenerating gas feed and the means for discharging the regeneration effluent; these means being adapted further to connect the regenerating tubes alternately to the regenerating gas feed and regeneration effluent discharge then to the charge-feeding means and the means for recovering the hydrocarbon effluent; the reaction tubes operating in so-called reaction phase while the regenerating tubes operate in so-called regeneration phase during a first stage, and the reaction tubes then becoming regeneration tubes while the regeneration tubes become reaction tubes during a second stage.

17. The apparatus of claim 16, wherein the convection chamber 13 has a heat exchanger adapted to preheat the charge, the regenerating gases and the purging gas.

18. The apparatus of any of claims 11 to 17, wherein the reaction and regeneration tubes are heated by said heating means arranged outside the latter.

19. The apparatus of any of claims 11 to 18, wherein the tubes comprise an annular portion containing the catalyst and a central, cylindrical portion inside which the heating means are housed.

20. Use of the apparatus of any of claims 11 to 19 in a method of producing olefinic hydrocarbons from a charge comprising at least one hydrocarbon with 2 to 20 carbon atoms which can be dehydrogenated.

## Patentansprüche

1. Verfahren zum Herstellen olefinischer Kohlenwasserstoffe aus einer Charge, die aliphatische Kohlenwasserstoffe mit 2 bis 20 Kohlenstoffatomen umfaßt, die geeignet sind, in wenigstens einem Reaktionsgefäß, das eine Vielzahl von im wesentlichen parallelen Rohren umfaßt, die in Reihen angeordnet sind und ein festes Katalysatorbett enthalten, dehydriert zu werden, bei dem man durchführt:
a) eine Reaktionsphase zur Erzeugung olefinischer Kohlenwasserstoffe, während der man diese gegebenenfalls vorgewärmte Charge in diesen die katalytische Zusammensetzung enthaltenden Rohren unter geeigneten Bedingungen zirkulieren läßt und einen an olefinischen Kohlenwasserstoffen reichen Abstrom sammelt;
b) eine Reinigungsphase der Rohre mit wenigstens einem geeigneten Gas nach der Reaktionsphase und nach einer Phase der Regeneration des unten definierten Katalysators, und man einen Reinigungsabstrom sammelt;
c) und eine Regenerationsphase in diesen Rohren des Gefäßes des Katalysator im festen Bett, auf dem Koks während der Reaktionsphase unter geeigneten Regenerationsbedingungen abgeschieden wird, und man einen Regenerationsabstrom gewinnt, wobei dieses Verfahren sich dadurch auszeichnet, daß man während der Reaktionsphase die Erwärmung der Rohre durch eine Vielzahl von geeigneten Strahlungsheizmitteln vornimmt, die in im wesentlichen parallelen Bahnen unabhängig voneinander angeordnet sind und die im wesentlichen senkrecht zu den Rohren verlaufen, wobei diese Heizmittel so ausgelegt sind, daß sie einen ersten Teil (Speiseseite) der Rohre, die 1 bis 50 % der Länge der Rohre darstellen, mit einem thermischen Strom erwärmen, der zwischen 101 und 500 % des thermischen mittleren Stroms des Reaktionsgefäßes darstellt und den verbleibenden Teil der Rohre mit einem thermischen Strom erwärmt, der zwischen 10 und 100 % des thermischen mittleren Flusses derart beträgt, daß die Isothermizität des Katalysators im wesentlichen aufrechterhalten wird und man gegebenenfalls aus diesem Gefäß Verbrennungsrauchgase, die aus diesen Heizmitteln stammen, abzieht.

2. Verfahren nach Anspruch 1, bei dem man 1 bis 40 % und bevorzugt 1 bis 35 % der Länge der Reaktionsrohre auf der Speiseseite mit einem thermischen Fluß erwärmt, der zwischen 120 und 300 %, und bevorzugt zwischen 150 und 200 % dieses thermischen mittleren Flußes beträgt und der verbleibende Teil der Reaktionsrohre mit einem thermischen Fluß erwärmt, der zwischen 20 und 85 %, und bevorzugt zwischen 40 und 75 % des mittleren thermischen Flußes umfaßt.

3. Verfahren nach einem der Ansprüche 1 bis 2, bei dem man zwischen jeder Reaktionsphase und jeder Regenerationsphase und zwischen jeder Regenerationsphase und jeder Reaktionsphase die Speisung jeweils mit Charge und Regenerationsgas stoppt; man stoppt gegebenenfalls die Speisung der Brenner mit Brennstoff und Verbrennungsluftträger und man reinigt mindestens einen Teil der Reaktionsrohre mit einem Inertgas wie Stickstoff unter Bedinungungen von Durchsatz und Temperatur, derart, daß die Temperatur des Katalysators im wesentlichen konstant ist.

4. Verfahren nach einem der Ansprüche 1 bis 3, bei dem das Reaktionsgefäß wenigstens einen Modul aus zwei Rohranordnungen umfaßt, dadurch gekennzeichnet, daß man die Reaktionsphase in einer dieser Rohranordnungen durchführt, während man die Regenerationsphase des Katalysators in der anderen Rohranordnung und umgekehrt durchführt.

5. Verfahren nach einem der Ansprüche 1 bis 4, dadurch gekennzeichnet, daß die Regenerationsphase des Katalysators die Injektion eines Inertgases wie Stickstoff, gegebenenfalls vorgewärmt, und wenigstens eines Gases umfaßt, das zwischen 0,01 und 5 % Sauerstoff-Molvolumen in diesen Reaktionsrohren enthält und die Erwärmung zwischen 1 und 50 %, und bevorzugt zwischen 1 und 35 % der Länge der Reaktionsrohre speiseseitig mit einem thermischen Fluß zwischen 5 und 100 % des mittleren thermischen Flusses des Gefäßes unter Bedingungen derart umfaßt, daß die Exothermizität der Verbrennungsreaktion des Kokses geregelt und die Temperatur des Katalysators im wesentlichen konstant wird.

6. Verfahren nach einem der Ansprüche 1 bis 5, bei dem man eine Oxychlorierungsstufe mittels eines Gases, das molekularen Sauerstoff und Chlor enthält oder mittels einer chlorierten Zusammensetzung bei einer Temperatur zwischen 450 und 500 °C, dann gegebenenfalls eine Kalzinierungsstufe mit einer höheren Sauerstoffkonzentration als der in Anspruch 6 beschriebenen, durchführt.

7. Verfahren nach einem der Ansprüche 1 bis 6, bei dem die Charge frischen oder rezyklierten Wasserstoff umfaßt.

8. Verfahren nach einem der Ansprüche 1 bis 7, bei dem die Charge Wasserdampf umfaßt.

9. Verfahren nach einem der Ansprüche 1 bis 8, bei dem die Heizmittel Brenner mit Matrix aus Keramikfaser sind.

10. Verfahren nach einem der Ansprüche 1 bis 9, bei dem man die Isothermizität des Katalysators durch Behandlungs- und Steuermittel regelt und einstellt, die mit den Strahlungsheizmitteln verbunden sind und mit wenigstens einer ersten Temperatursonde, die mit dem ersten Teil der Rohre und mit wenigstens einer zweiten Sonde verbunden ist, die mit dem verbleibenden Teil der Rohre verbunden sind.

11. Verfahren zum Umwandeln von Kohlenwasserstoffen, wenigstens ein erstes Gefäß (1) umfassend, das wenigstens einen Reaktor (2) enthält, in dem eine endotherme Reaktion durchgeführt wird und der eine Vielzahl von im wesentlichen untereinander parallelen Rohren (3) umfaßt, die parallel gespeist sind, wobei die Rohre mit wenigstens einem Katalysator im festen Bett gefüllt sind, Mittel 4 zum Speisen mit einer Charge, die mit einem Ende dieser Rohre verbunden sind und Gewinnungsmittel (5) eines Kohlenwasserstoffabstroms, die mit dem anderen Ende dieser Rohre verbunden sind, wobei diese Rohre in im wesentlichen parallelen Reihen angeordnet und unter geeigneten Bedingungen durch eine Vielzahl von Heizmitteln (6) erwärmt sind, wobei diese Heizmittel eine Speisung (7) mit Sauerstoffträger und Brennstoff und einen Abzug (16) für die Verbrennungsrauchgase, verbunden mit diesem Reaktor, umfassen, wobei die Vorrichtung im übrigen Regenerierungsmittel (9, 28) des verbrauchten Katalysators umfaßt, Reinigungsmittel der Rohre mittels wenigstens eines geeigneten Gases umfassend, Mittel (9a, 9b) zum Speisen mit wenigstens einem Regenerierungsgas sowie Mittel (10, 32) zum Abzug des Regenerierungsabstroms, die so eingerichtet sind, daß sie den verbrauchten Katalysator in diesen Rohren regenerieren, wobei diese Regenerierungsmittel mit diesen Rohren verbunden sind, sowie Mittel (20) zum alternativen Wechsel, die so ausgebildet sind, daß sie diese Rohre alternativ mit den Mitteln (4) zum Speisen mit der Charge und mit den Mitteln (5) zur Gewinnung des Abstroms und dann mit den Regenerierungsmitteln (9, 28) des Katalysators verbinden, wobei die Vorrichtung sich dadurch auszeichnet, daß sie Strahlungsheizmittel wie Brenner mit Keramikfasern umfaßt, die in im wesentlichen parallelen Bahnen unabhängig voneinander angeordnet sind und welche im wesentlichen senkrecht zu den Rohren verlaufen und daß sie im übrigen Mittel (50) zum Regeln und Einstellen der Temperatur der mit diesen Heizmittelbahnen verbundenen Rohren umfaßt und so ausgelegt sind, daß sie einen ersten Teil der Rohre speiseseitig mit einem thermischen Fluß größer als dem mittleren thermischen Fluß des Gefäßes erwärmen und den verbleibenden Teil der Rohre mit einem thermischen Fluß erwärmen, der höchstens gleich dem mittleren thermischen Fluß des Gefäßes ist.

12. Vorrichtung nach Anspruch 11, bei der die Länge der Rohre zwischen 2 und 20 m und deren Innendurchmesser zwischen 10 und 200 mm beträgt.

13. Vorrichtung nach einem der Ansprüche 11 bis 12, bei der der Zwischenachsabstand jedes Rohres zwischen dem 1,5 und 6-fachen seines Durchmessers beträgt.

14. Vorrichtung nach einem der Ansprüche 11 bis 13, bei der die Regel- und Einstellmittel wenigstens eine Temperatursonde umfassen, die mit einem ersten Teil der Rohre speiseseitig und wenigstens eine andere Sonde umfaßt, die mit einem zweiten nachfolgenden Teil der Rohre verbunden ist, wobei diese beiden Sonden Signale liefern, Verarbeitungs- und Steuermittel derartiger Einrichtung, daß sie die Signale empfangen, sie mit Bezugssignalen vergleichen und andere Signale übertragen, die in der Lage sind, die Heizmittel des ersten und zweiten Teils der Rohre zu steuern.

15. Vorrichtung nach einem der Ansprüche 11 bis 14, bei der der Abstand zwischen den Heizmitteln zwischen 0,5 und 2,0 m beträgt.

16. Vorrichtung nach einem der Ansprüche 11 bis 15, bei der dieses Gefäß einen ersten Reaktor, der eine Vielzahl von Reaktionsrohren enthält und einen zweiten Reaktor umfaßt, der eine Vielzahl von Regenerationsrohren einer zur Regenerierung des Katalysators geeigneten Art umfaßt, wobei der erste und der zweite Reaktor über wenigstens einen Durchlaß (14, 15) mit einer Konvektionskammer (13) kommuniziert, die mit der äußeren Umgebung über geeignete Mittel (Kamin) verbunden ist, wobei diese Regenerationsrohre an einem Ende mit einer Speisung mit Regenerationsgas und das andere Ende mit einem Abzug eines Regenerationsabstroms verbunden sind und im übrigen Mittel umfassen, die so eingerichtet sind, daß sie die Reaktionsrohre alternativ mit den Speisemitteln und mit den Mitteln zum Gewinnen des Abstroms, dann mit der Speisung mit Regenerationsgas und mit dem Abzug des Regenerationsabstroms verbinden, wobei diese Mittel so eingerichtet sind, daß sie im übrigen die Regenerationsrohre alternativ mit der Regenerationsgasspeisung und mit dem Abzug des Regenerationsabstroms, dann mit den Mitteln der Chargenspeisung und mit den Gewinnungsmitteln des Kohlenwasserstoffabstroms verbinden, wobei die Reaktionsrohre in der sogenannten Reaktionsphase arbeiten, während die Regenerationsrohre in der sogenannten Regenerationsphase in einem ersten Takt arbeiten und die Reaktionsrohre dann zu Regenerationsrohren werden, während die Regenerationsrohre Reaktionsrohre in einem zweiten Takt werden.

17. Vorrichtung nach Anspruch 16, bei der die Konvektionskammer (13) einen Wärmeaustauscher umfaßt, der so eingerichtet ist, daß er die Charge, die Regenerationsgase und das Reinigungsgas vorwärmt.

18. Vorrichtung nach einem der Ansprüche 11 bis 17, bei der die Reaktions- und Regenerationsrohre durch diese außerhalb von diesen angeordneten Heizmittel erwärmt werden.

19. Vorrichtung nach einem der Ansprüche 11 bis 18, bei der die Rohre einen den Katalysator enthaltenden Ringteil und einen zentralen zylindrischen Teil umfassen, innerhalb dessen diese Heizmittel gelagert sind.

20. Verwendung der Vorrichtung nach einem der Ansprüche 11 bis 19 in einem Verfahren zur Erzeugung olefinischer Kohlenwasserstoffe ausgehend von einer Charge, die wenigstens einen dehydrierbaren Kohlenwasserstoff mit 2 bis 20 Kohlenstoffatomen umfaßt.
